# EUROPEAN PATENT SPECIFICATION

(11) **EP 2 073 863 B1**
(45) Date of publication and mention of the grant of the patent: **19.10.2011**
(21) Application number: 07802801.6
(22) Date of filing: 22.08.2007
(51) Int. Cl.: A61M 1/36

(54) **A FILTER FOR THE REMOVAL OF SUBSTANCES FROM BLOOD PRODUCTS**
FILTER ZUR ENTFERNUNG VON SUBSTANZEN AUS BLUTPRÄPARATEN
FILTRE PERMETTANT DE RETIRER DES SUBSTANCES DE PRODUITS SANGUINS

(30) Priority: 06.09.2006 EP 06425617
(43) Date of publication of application: 01.07.2009
(73) Proprietor: Fresenius Hemocare Italia S.r.l., I-41032 Cavezzo (Modena) (IT)
(72) Inventor: BERTOLUCCI, Massimo, I-55049 Viareggio (Lucca) (IT); BONAGUIDI, Paolo, I-56123 Pisa (IT); BRUINI, Marco, I-41030 Quarantoli (Modena) (IT)
(74) Representative: Grund, Martin
(86) International application number: PCT/EP2007/058729
(87) International publication number: WO 2008/028807

(56) References cited:
- WO-A-2005/113136
- US-A1- 2003 158 364

## Description

The present invention relates to a filter for the removal of substances from blood or blood components and more particularly to a filter for leukocytes and platelets removal, adapted for use in blood purification devices, such as blood bag systems which are conventionally used for the separation of whole blood into leukocyte depleted hemocomponents.

The blood filters, to which the present invention relates, typically comprise a housing with inlet and outlet ports and at least a porous element within the housing, interposed between the inlet and the outlet port. The porous element usually consists of a web, which may be formed by one or more layers of filtering material, typically a non-woven fabric made of polymeric fibre material.

Polyesters, such as polybutylentherephthalate (PBT) and polyethylenetherephthalate (PET), constitute a currently preferred polymeric material. It is known that non-woven fabrics made of polyesters like PET or PBT fibres can strongly interact with platelets; therefore, their use in blood filtration would lead to a high rate of platelet removal. On the other hand, in filtration experiments, it is also evident that said polymers have a low ability of removing leukocytes and their low wettability decreases the bleeding rate of the hemocomponents and increases the priming time. Surface modification of polyester fibres is therefore highly preferred in blood filtration devices.

In order to increase the wettability or the Critical Wetting Surface Tension (CWST), which is a measure of the hydrophilicity of the material, fibres of a hydrophobic resin, such as PBT, have been coated with a more hydrophilic polymer, including hydrophilic polyurethanes.

Polyurethanes (PU) are widely employed as biocompatible polymers. In particular, segmented polyurethanes with a polyethylene glycol chain as the soft block are recognised as biocompatible polymers inducing low thrombogenicity.

It is known that the wettability of a segmented polyurethane containing a polyethylene glycol (PEG) soft segment can be tuned by modification of the PEG content in the polymer. On the other hand, such an enhancement of wettability is often coupled with an increase of water leachates. Moreover, more wettable materials can also induce an increase of the platelet compatibility and therefore a reduction of their removal from the blood component being filtered.

WO2005/113136 describes a polyurethane material with improved leukocyte adsorption capacity, which is composed of a diisocyanate compound structural unity, a polymer diol compound structural unity and a chain extender structural unity, preferably containing a tertiary amino group. According to the description and claims, the polyurethane must have a weight average molecular weight equal to or more than 20,000 and not more than 1,000,000, since the water solubility of such polyurethanes with a weight average molecular weight lower than 20,000 is reported to be insufficiently low for using the polyurethane as a leukocyte filter material.

According to the present invention, it has been found that - contrary to the teaching of WO2005/113136 polyurethanes obtained by the addition reaction of a diisocyanate, a polymer diol (such as polyethylene glycol) and a monomer diol exhibit the best compromise of those properties which are required for their use as a suitable material for blood filters, if their number average molecular weight is not higher than 10,000 Da.

Particularly, the polyurethanes of the invention allow to optimise different and diverging needs, such as:
- the best compromise in terms of leukocytes and platelets removal;
- the absence of relevant water leachates;
- a good wettability to ensure the hemocomponent bleeding through the filter;
- a low priming time.

The subject matter of the invention is therefore a filter for the removal of substances from blood or blood components including a filter material as defined in the appended claims.

The subject matter of the invention is also directed to the use of the filter for the depletion of leukocytes and platelets from either whole blood or blood components in ex vivo procedures, as well as blood purification systems and devices comprising such a filter, as defined in the appended claims.

The term "polyurethane" as used herein includes polyurethane copolymers.

The diisocyanate, which is used for the preparation of the polyurethane, is preferably an aliphatic diisocyanate, such as particularly isophorone diisocyanate (IPDI), hexamethylene diisocyanate (HDI), dicycloexyl methane diisocyanate (HMDI), trimethylhexamethylene diisocyanate, 1,3-bis(isocyanomethyl) cycloexane and diisocyanates of dimer acids.

The polymer diol is preferably polyethylene glycol, although other polymer diols, such as polypropylene glycol, polytetramethylene glycol, poly(ethylene glycol/propylene glycol) copolymer, poly(ethylene glycol/tetramethylene glycol) copolymer can be used.

Polymeric diols and particularly polyethylene glycol having a number average molecular weight not higher than 400 Da are preferred.

The monomer diol is preferably butandiol, particularly 1,4-butane diol, although in general monomeric diol compounds having from 2 to 6 carbon atoms can be used.

The polyurethane which is used according to the invention has an average number molecular weight lower than 10,000 Dalton; preferably also the weight average molecular weight is lower than 10,000 Dalton. A low degree of polymerisation is obtained by a deliberate unbalance of the stoichiometry in the polyurethane system. In a stoichiometrically balanced polyurethane, the number (equivalents) of isocyanate groups, in the addition reaction, is approximately equal to the sum of the hydroxyl groups of the polymeric diol (PEG) and of the monomer diol (butandiol).

On the other hand, the stoichiometrically unbalanced polyurethanes are obtained by using in the reaction a stoichiometric excess of the monomer diol, whereby the total number (equivalents) of the hydroxyl groups provided in the reaction by the polymeric diol and monomer diol is in excess in respect of the number (equivalents) of the isocyanate groups.

Thus, the invention relates to a filter for the removal of substances from blood or blood components, comprising a polymeric filter material having a coating comprising polyurethane, characterised in that said polyurethane has a number average molecular weight not higher than 10,000 Da, wherein said polyurethane is obtained by the addition reaction of a diisocyanate, a polymer diol and a monomer diol and by unbalancing the stoichiometry of the addition reaction by adding an excess of the monomer diol.

The polyurethanes can be synthesised by conventional procedures. According to the preferred process, the diisocyanate is first reacted with an amount of the polymeric diol (PEG) in stoichiometric defect, in a suitable organic solvent, such as anhydrous methyl ethyl ketone, at reflux temperature, in the presence of a catalyst, such as dibutyltin diacetate; the reaction mixture is brought to the reflux temperature and allowed to react; then, the monomer diol (butandiol) is added in a stoichiometric excess and the reaction mixture is again allowed to react at reflux temperature, until exhaustion of the isocyanate functionality.

The mixture is then allowed to cool down at room temperature and the obtained polymer is precipitated by dropping the reaction mixture into a non-polar solvent, such as hexane. The resulting precipitate is then dried and dissolved in a polar solvent, such as acetone. In order to improve the purification, the polymer may be subjected to a further precipitation in water. In particular, a double precipitation of the polyurethanes into water ensures that the polymers are not water soluble and that any water soluble matter is strongly reduced.

Preferably, the polyurethanes which are used according to the invention have a polymer diol content (preferably PEG) not higher than 35% by weight; due to their hydrophilichydrophobic balance and also owing to the preferred purification procedure, the synthesised polyurethanes are not water soluble.

In the preferred embodiment of the invention, the polyurethanes are applied as coatings on non-woven polyester fabrics, obtained from melt-blown fibres of PET or PBT. The coatings may be carried out according to the usual solution coating technique, as described in the wettability tests which follow.

The interaction between the coating and the substrate (namely PBT) is likely to be due only to hydrophobic interactions and no covalent bonding between the coating and the substrate appears to be involved in any stage of the coating.

The invention is further illustrated by the following non-limiting examples. Examples 1 and 2 relate to the preparation of stoichiometrically unbalanced polyurethanes (identified as UB polymers) for use in the filter of the invention; examples 3-6 are comparative examples relating to stoichiometric polyurethanes (identified as ST polymers).

### EXAMPLES

### Example 1

A reaction flask, equipped with an addition funnel and a reflux condenser, is charged with 300 ml of anhydrous butanone (MEK), 21.80 g of PEG with average molecular weight 204.4 Da (0.107 mol), 48.06 g of isophorone diisocyanate (0.216 mol) and 0.079 g of dibutyltin diacetate. The reaction mixture was gently heated to the reflux (internal temperature is about 82°C). After two hours, the heating was interrupted and a solution of 10.98 g (0.122 mol) of butanediol in 35 ml of MEK was added dropwise. The heating was then restarted till the reflux of the mixture was again reached. After 15 hours, the absence of the isocyanate band in the IR spectrum was checked to assess the completion of the reaction. The mixture was allowed to cool down to room temperature and the polymer precipitated by dropping the reaction mixture into 800 ml of hexane. The resulting precipitate was dried and dissolved in acetone to yield a 20% solution. To obtain a more purified polymer, the polymer (hereinafter UB 15) was precipitated into 2.2 ℓ of water and then dried.

### Example 2

A reaction flask, equipped with an addition funnel and a reflux condenser, is charged with 300 ml of anhydrous butanone (MEK), 23.11 g of PEG with average molecular weight 204.4 Da (0.113 mol), 50.05 g of isophorone diisocyanate (0.225 mol) and 0.073 g of dibutyltin diacetate. The reaction mixture was gently heated to the reflux (internal temperature is about 82°C). After two hours, the heating was interrupted and a solution of 11.72 g (0.130 mol) of butanediol in 40 ml of MEK was added dropwise. The heating was then restarted till the reflux of the mixture was again reached. After 15 hours, the absence of the isocyanate band in the IR spectrum was checked to assess the completion of the reaction. The mixture was allowed to cool down to room temperature and the polymer precipitated by dropping the reaction mixture into 800 ml of hexane. The resulting precipitate was dried and dissolved in acetone to yield a 20% solution. To obtain a more purified polymer, the polymer (hereinafter UB20) was precipitated into 2.2 ℓ of water and then dried.

### Example 3 - Comparative

A reaction flask, equipped with an addition funnel and a reflux condenser, is charged with 620 ml of anhydrous butanone (MEK), 46.51 g of PEG with average molecular weight 202.2 Da (0.230 mol), 100 g of isophorone diisocyanate (0.450 mol) and 0.16 g of dibutyltin diacetate. The reaction mixture was gently heated to the reflux (internal temperature is about 82°C). After two hours, the heating was interrupted and a solution of 19.83 g (0.220 mol) of butanediol in 60 ml of MEK was added dropwise. The heating was then restarted till the reflux of the mixture was again reached. After 15 hours, the absence of the isocyanate band in the IR spectrum was checked to assess the completion of the reaction. The mixture was allowed to cool down to room temperature and the polymer precipitated by dropping the reaction mixture into 4 ℓ of deionised water. The resulting precipitate was dried and dissolved in acetone to yield a 20% solution. To obtain a more purified polymer, the polymer (hereinafter ST27) was again precipitated into water (water/polymer solution = 5/1 by weight) and then dried.

### Example 4 - Comparative

A reaction flask, equipped with an addition funnel and a reflux condenser, is charged with 300 ml of anhydrous butanone (MEK), 29.17 g of PEG with average molecular weight 204.4 Da (0.143 mol), 44.88 g of isophorone diisocyanate (0.202 mol) and 0.079 g of dibutyltin diacetate. The reaction mixture was gently heated to the reflux (internal temperature is about 82°C). After two hours, the heating was interrupted and a solution of 4.94 g (0.055 mol) of butanediol in 30 ml of MEK was added dropwise. The heating was then restarted till the reflux of the mixture was again reached. After 15 hours, the absence of the isocyanate band in the IR spectrum was checked to assess the completion of the reaction. The mixture was allowed to cool down to room temperature and the polymer precipitated by dropping the reaction mixture into 800 ml of hexane. The resulting precipitate was dried and dissolved in acetone to yield a 20% solution. To obtain a more purified polymer, the polymer (hereinafter ST37) was precipitated into 2.2 ℓ of water and then dried.

### Example 5 - Comparative

A reaction flask, equipped with an addition funnel and a reflux condenser, is charged with 300 ml of anhydrous butanone (MEK), 34.95 g of PEG with average molecular weight 204.4 Da (0.171 mol), 42.05 g of isophorone diisocyanate (0.189 mol) and 0.079 g of dibutyltin diacetate. The reaction mixture was gently heated to the reflux (internal temperature is about 82°C). After two hours, the heating was interrupted and a solution of 1.3 g (0.014 mol) of butanediol in 30 ml of MEK was added dropwise. The heating was then restarted till the reflux of the mixture was again reached. After 15 hours, the absence of the isocyanate band in the IR spectrum was checked to assess the completion of the reaction. The mixture was allowed to cool down to room temperature and the polymer precipitated by dropping the reaction mixture into 800 ml of hexane. The resulting precipitate was dried and dissolved in acetone to yield a 20% solution. To obtain a more purified polymer, the polymer (hereinafter ST45) was precipitated into 2.2 ℓ of water and then dried.

### Example 6 - Comparative

A reaction flask, equipped with an addition funnel and a reflux condenser, is charged with 130 ml of anhydrous butanone (MEK), 10.61 g of PEG with average molecular weight 403.4 Da (0.026 mol), 18.00 g of isophorone diisocyanate (0.081 mol) and 0.043 g of dibutyltin diacetate. The reaction mixture was gently heated to the reflux (internal temperature is about 82°C). After two hours, the heating was interrupted and a solution of 4.93 g (0.055 mol) of butanediol in 40 ml of MEK was added dropwise. The heating was then restarted till the reflux of the mixture was again reached. After 15 hours, the absence of the isocyanate band in the IR spectrum was checked to assess the completion of the reaction. The mixture was allowed to cool down to room temperature and the polymer precipitated by dropping the reaction mixture into 800 ml of hexane. The resulting precipitate was dried and dissolved in acetone to yield a 20% solution. To obtain a more purified polymer, the polymer (hereinafter ST30-400) was precipitated into 2.2 of water and then dried.

The molecular weights of all the prepared polymers were determined by GPC analysis.

A Shimadzu HPLC system 10 Avp equipped with a PL-Gel 5 µm MIXED-D column and a R.I. detector was used for the analysis. GPC determinations were performed at 30°C, using tetrahydrofuran as the mobile phase; the system was calibrated against monodispersed polystyrene standards.

The results of the analysis are summarised in Table 1.

**TABLE I**

| Polymer | %PEG¹ | Mₙ (Da) | M_{w} (Da) | M_{w}/Mₙ |
|---|---|---|---|---|
| UB15 | 27 | 3450 | 8412 | 2.4 |
| UB20 | 27 | 3310 | 7570 | 2.3 |
| ST27 | 27 | 56199 | 156529 | 2.7 |
| ST37 | 37 | 14500 | 29783 | 2.0 |
| ST45 | 45 | 14939 | 30580 | 1.2 |
| ST-30-400 | 32 | 18905 | 41395 | 2.2 |

| | | | | |
|---|---|---|---|---|
| ¹polyethylene glycol (wt% in the feed) | | | | |

### EVALUATION OF WATER LEACHATES

The coated PBT fabric which is used in the filter according to the invention was also successfully tested for the possible removal of water soluble species, according to the method described in the following example for the evaluation of water leachates, which is a modification of the method 3.2.6 of Ph. Eur., as indicated by the ISO EN 10993/12.

For the evaluation of water leachates, 40 layers of the fabric with a surface area of 50 cm² (coated with a solution of 0.5% of the polyurethane in acetone) are introduced in a housing having an inlet and an outlet port.

250 ml of distilled water was circulated through one filter employing a peristaltic pump at a flow rate of 1 1/h for two hours at 37°C. The obtained solution was analysed as follows.

The absorbance of the solution was measured between 230 and 360 nm.

20 ml of the solution was reacted with 20 ml of 0.01 N potassium permanganate in presence of sulphuric acid at boiling temperature for 3 minutes. After cooling down the solution, an excess of potassium iodide was added and iodine formed was titrated with sodium thiosulphate 0.01 N. A blank solution was treated in the same way. The difference between the permanganate titration volumes (ΔOx, ml) is calculated.

The weight of the dry solid residue was measured: 100 ml of the solution was evaporated and the residue dried at 100-105°C to constant weight.

For all the tested polymers, the measured absorbance was lower than 0.2 in the whole examined range, ΔLOx < 1 ml and the dry solid residue < 3 mg.

### WETTABILITY TESTS

All the synthesised materials were used to coat a PBT based non-woven fabric. The fabric was immersed in a solution of the polymer in acetone, squeezed to remove excess solution and then dried for 16 h at 70°C in air.

Water wettability of the coated fabric was evaluated by placing five drops of water on the fabric and measuring the time required for the complete absorption of the drop by the fabric. The coating solution for the wettability measurement had a concentration of the polymer of 2% wt.

The absorption times are reported in Table II.

**TABLE II**

| Polymer | PBT | UB15 | UB20 | ST27 | ST37 | ST45 | ST30-400 |
|---|---|---|---|---|---|---|---|
| Time (seconds) | >600 | 227 | 107 | >600 | 490 | 73 | 10 |

### BLOOD FILTRATION TESTS

Whole blood (range 450-550 ml) was collected from healthy random volunteers in PVC bags with 70 ml of CPD.

### Small scale filtrations

2% coated and non-coated PBT non-woven fabric samples were punch cut into 3 cm diameter circles and placed in a holder with an inlet and an outlet that allows the passage of the blood through the fabric.

2 ml of human whole blood was filtered through the device containing three layers of the non-woven fabric.

The cellular content of each sample was determined by means of an automated cell counter.

The reported results (Table III) are an average of at least five filtration experiments.

**TABLE III**

| Polymer | PLT removal % | WBC removal % |
|---|---|---|
| NT | 48 | 79 |
| UB15 | 34 | 92 |
| UB20 | 35 | 92 |
| ST27 | 31 | 81 |
| ST45 | 27 | 90 |
| ST30-400 | 20 | 92 |

It is evident from the reported data that the best compromise of platelet (PLT) and leukocyte (WBC) removal is obtained by the fabric coated with the UB series polyurethanes.

### BLOOD-BANK-SCALE FILTRATION

Preparation of the filters: 40 layers of the fabric with a surface area of 50 cm² (coated with a solution of 0.5% of the polyurethane in acetone or uncoated) were placed in a housing having an inlet and an outlet port to allow blood filtration under the effect of gravity.

All whole-blood donations were cooled at 20-24°C under 1,4-butane-diol plates. The cell concentration was determined by an automated cell counter in the blood, before the filtration and after the filtration, in order to determine the total platelet (PLT) removal %. White blood cells (WBCs) in the filtered blood were counted in a Nageotte cell.

The obtained results are summarised in Table IV.

**TABLE IV**

| Run | Filter fabric | Filtration time (min) | PLT removal % | Residual WBC/unit x 10⁶ |
|---|---|---|---|---|
| 1 | Untreated PBT | 30 | 99 | 0.70±0.5 |
| 2 | UB15 | 14 | 100 | 0.32 ±0.2 |
| 3 | UB20 | 14 | 100 | 0.19±0.1 |
| 4 | ST45 | 16 | 75 | 0.18 ±0.3 |
| 5 | ST27 | 20 | 98 | 0.52 ±0.5 |

It is therefore confirmed that the use of the UB series polyurethane enhances the filtration performance (cell removal and filtration time) of the coated fabric.

## Claims

1. A filter for the removal of substances from blood or blood components, comprising a polymeric filter material having a coating comprising polyurethane, **characterised in that** said polyurethane has a number average molecular weight not higher than 10,000 Da, wherein said polyurethane is obtained by the addition reaction of a diisocyanate, a polymer diol and a monomer diol and by unbalancing the stoichiometry of the addition reaction by adding an excess of the monomer diol.

2. A filter according to claim 1, wherein the diisocyanate is an aliphatic diisocyanate, preferably isophorone diisocyanate.

3. A filter according to claims 1 or 2, wherein the polymer diol has a number average molecular weight not higher than 400 Da.

4. A filter according to any of claims 1 to 3, wherein said polyurethane has a content of polymer diol not higher than 35% by weight.

5. A filter according to any of claims 1 to 4, wherein the polymer diol is polyethylene glycol.

6. A filter according to any of claims 1 to 5, wherein the monomer diol is butandiol.

7. A filter according to any of the preceding claims, wherein said polymeric filter material comprises a non-woven polyester fabric coated with said polyurethane coating.

8. A filter according to claim 7, wherein said non-woven polyester fabric comprises melt-blowN polybuthylenetherephthalate or polyethylenetherephthalate fibres.

9. A filter according to any of the preceding claims, wherein said polyurethane has a weight average molecular weight not higher than 10,000 dA.

10. The use of a filter according to any of claims 1 to 9 for the depletion of leukocytes and platelets from whole blood or blood components in ex vivo filtration procedures.

11. A blood purification device comprising a filter according to any of claims 1 to 9.

12. A blood purification device according to claim 11, comprising a blood bag device for the separation of blood into leukocyte depleted blood components, comprising a first bag connected, in fluid flow communication, with a second bag through a leukocyte filter according .to any of claims 1 to 9.

13. A method for producing a filter according to any of claims 1 to 9, comprising coating a porous substrate consisting of a non-woven fabric of a polyester fibre material with a polyurethane coating, as described in any of claim 1 to 9.

## Patentansprüche

1. Ein Filter zur Entfernung von Substanzen aus Blut oder Blutkomponenten, umfassend ein polymeres Filtermaterial mit einer Beschichtung umfassend Polyurethan, charakterisiert **dadurch**, dass das Polyurethan ein zahlengemitteltes Molekulargewicht nicht höher als 10000 Da hat, wobei das Polyurethan erhalten wird durch die Additionsreaktion eines Diisocyanats, eines polymeren Diols und eines monomeren Diols und durch das aus dem Gleichgewicht Bringen der Stöchiometrie der Additionsreaktion durch die Zugabe eines Überschusses des monomeren Diols.

2. Ein Filter gemäß Anspruch 1, wobei das Diisocyanat ein aliphatisches Diisocyanat, vorzugsweise Isophorondiisocyanat, ist.

3. Ein Filter gemäß Ansprüchen 1 oder 2, wobei das polymere Diol ein zahlengemitteltes Molekulargewicht nicht höher als 400 Da hat.

4. Ein Filter gemäß irgendeinem der Ansprüche 1 bis 3, wobei das Polyurethan einen Gehalt an polymerem Diol nicht höher als 35 Gewichtprozent hat.

5. Ein Filter gemäß irgendeinem der Ansprüche 1 bis 4, wobei das polymere Diol Polyethylenglykol ist.

6. Ein Filter gemäß irgendeinem der Ansprüche 1 bis 5, wobei das monomere Diol Butandiol ist.

7. Ein Filter gemäß irgendeinem der vorhergehenden Ansprüche, wobei das polymere Filtermaterial einen nichtgewebten Polyesterstoff beschichtet mit der Polyurethanbeschichtung umfasst.

8. Ein Filter gemäß Anspruch 7, wobei der nichtgewebte Polyesterstoff schmelzgeblasene Polybutylenterephthalat- oder Polyethylenterephthalatfasern umfasst.

9. Ein Filter gemäß irgendeinem der vorhergehenden Ansprüche, wobei das Polyurethan ein gewichtsgemitteltes Molekulargewicht nicht höher als 10000 dA hat.

10. Die Verwendung eines Filters gemäß irgendeinem der Ansprüche 1 bis 9 zur Depletion von Leukozyten und Plättchen aus Vollblut oder Blutkomponenten in *ex-vivo-*Filterverfahren.

11. Eine Blutreinigungsvorrichtung umfassend einen Filter gemäß irgendeinem der Ansprüche 1 bis 9.

12. Eine Blutreinigungsvorrichtung gemäß Anspruch 11, umfassend eine Blutbeutelvorrichtung zur Trennung von Blut in leukozytendepletierte Blutkomponenten, umfassend einen ersten Beutel, der in Flüssigkeitsfließverbindung über einen Leukozytenfilter gemäß irgendeinem der Ansprüche 1 bis 9 mit einem zweiten Beutel verbunden ist.

13. Ein Verfahren zur Herstellung eines Filters gemäß irgendeinem der Ansprüche 1 bis 9, umfassend Beschichtung eines porösen Substrates bestehend aus einem nichtgewebten Stoff aus einem Polyesterfasermaterial mit einer Polyurethanbeschichtung, wie in irgendeinem der Ansprüche 1 bis 9 beschrieben.

## Revendications

1. Filtre permettant de retirer des substances du sang ou de composants sanguins, comprenant un matériau polymère de filtration comportant un revêtement comprenant du polyuréthane, **caractérisé en ce que** ledit polyuréthane possède un poids moléculaire moyen en nombre d'au plus 10 000 Da, dans lequel ledit polyuréthane est obtenu par la réaction d'addition d'un diisocyanate, d'un diol polymère et d'un diol monomère et par le déséquilibre de la stoechiométrie de la réaction d'addition par l'ajout d'un excès du diol monomère.

2. Filtre selon la revendication 1, dans lequel le diisocyanate est un diisocyanate aliphatique, de préférence le diisocyanate d'isophorone.

3. Filtre selon la revendication 1 ou 2, dans lequel le diol polymère possède un poids moléculaire moyen en nombre d'au plus 400 Da.

4. Filtre selon l'une quelconque des revendications 1 à 3, dans lequel ledit polyuréthane présente une teneur en diol polymère d'au plus 35 % en poids.

5. Filtre selon l'une quelconque des revendications 1 à 4, dans lequel le diol polymère est un polyéthylène glycol.

6. Filtre selon l'une quelconque des revendications 1 à 5, dans lequel le diol monomère est le butanediol.

7. Filtre selon l'une quelconque des revendications précédentes, dans lequel ledit matériau polymère de filtration comprend un tissu non tissé de polyester revêtu avec ledit revêtement de polyuréthane.

8. Filtre selon la revendication 7, dans lequel ledit tissu non tissé de polyester comprend des fibres de poly(téréphtalate de butylène) ou de poly(téréphtalate d'éthylène) obtenues par fusion-soufflage.

9. Filtre selon l'une quelconque des revendications précédentes, dans lequel ledit polyuréthane possède un poids moléculaire moyen en poids d'au plus 10 000 Da.

10. Utilisation d'un filtre selon l'une quelconque des revendications 1 à 9, pour la réduction du nombre des leucocytes et des plaquettes contenus dans du sang entier ou dans des composants sanguins dans des procédures de filtration ex vivo.

11. Dispositif de purification du sang, comprenant un filtre selon l'une quelconque des revendications 1 à 9.

12. Dispositif de purification du sang selon la revendication 11, comprenant un dispositif pour poche de sang servant à séparer du sang en composants sanguins appauvris en leucocytes, comprenant une première poche raccordée, en communication de fluide, à une seconde poche par l'intermédiaire d'un filtre à leucocytes selon l'une quelconque des revendications 1 à 9.

13. Procédé de production d'un filtre selon l'une quelconque des revendications 1 à 9, comprenant le revêtement d'un substrat poreux constitué par un tissu non tissé de matériau de fibre de polyester avec un revêtement à base de polyuréthane, comme décrit dans l'une quelconque des revendications 1 à 9.
